# EUROPÄISCHE PATENTSCHRIFT

(11) **EP 1 893 090 B1**
(45) Veröffentlichungstag und Bekanntmachung des Hinweises auf die Patenterteilung: **12.09.2012**
(21) Anmeldenummer: 06742735.1
(22) Anmeldetag: 28.04.2006
(51) Int. Cl.: A61B 6/00, A61B 6/14

(54) **VERFAHREN UND SYSTEM ZUR KNOCHENDICHTEKALIBRIERUNG**
BONE DENSITY CALIBRATION METHOD AND SYSTEM
PROCEDE ET SYSTEME D'ETALONNAGE DE DENSITE OSSEUSE

(30) Priorität: 04.05.2005 DE 102005021327
(43) Veröffentlichungstag der Anmeldung: 05.03.2008
(73) Patentinhaber: siCAT GmbH & Co. KG, 53177 Bonn (DE)
(72) Erfinder: ZÜNDORF, Gerhard, 53121 Bonn (DE); SCHROEDER, Martin, 53175 Bonn (DE); RITTER, Lutz, 53332 Bornheim (DE)
(74) Vertreter: Braun-Dullaeus, Karl-Ulrich
(86) Internationale Anmeldenummer: PCT/EP2006/003982
(87) Internationale Veröffentlichungsnummer: WO 2006/117147

(56) Entgegenhaltungen:
- WO-A-03/071934
- US-A- 5 335 260
- US-A1- 2002 150 205
- US-A1- 2003 235 265
- US-B1- 6 190 042
- US-B1- 6 674 834

## Beschreibung

Die vorliegende Erfindung betrifft ein Verfahren zur bildlichen Darstellung und zur Bestimmung von Gewebsdichten eines Körperbereiches, insbesondere im Kieferbereich, eines Patienten, wobei mittels einer Bilderzeugung einerseits ein Satz dreidimensionaler Bilddaten, die den Körpererbereich repräsentieren, und andererseits als dreidimensionale Bilddaten vorliegende Kalibrierungsdaten, die ein Kalibrierungsphantom bekannter Dichte repräsentieren, aufgenommen werden, wobei aus einem Vergleich der Kalibrierungsdaten mit den Bilddaten auf die Gewebsdichten geschlossen wird. Die Erfindung betrifft gleichsam ein System zur Durchführung eines solchen Verfahrens.

Generell ist eine solche örtlich aufgelöste Bestimmung der Knochendichte insbesondere in der dentalen Implantologie von großem Interesse, da sie dazu beiträgt, bei der Planung der Zahnimplantate Fehler zu vermeiden. Oft wird die Planung eines Implantats anhand der dreidimensionalen Repräsentation eines menschlichen Mund-, Kiefer-, Gesichtsbereichs (nachfolgend vereinfachend Kieferbereich) vorgenommen, wobei der Repräsentation Daten zugrunde liegen, die beispielsweise mittels Computer Volumen Tomographie (DVT) oder mittels. Computertomographie (CT) erstellt wurden. Bei derartigen Repräsentationen entsprechen die Voxelwerte, also die Farbwerte der einzelnen Volumenelemente, den unterschiedlichen Materialeigenschaften des gescannten Volumens.

Bekanntermaßen liegen diese Werte jedoch zunächst nur als relative Größen vor und können erst anhand von Kalibrierungsdaten in absolute Größen umgerechnet werden. So wird die Kalibrierung der Bilddaten im Falle des CT in der Einheit "Hounsfield" vorgenommen. Die Verwendung der Hounsfieldeinheiten hat im Fall der Knochendichtebestimmung insofern gravierende Nachteile, als diese durch viele Faktoren beeinflusst werden. So sind die Hounsfieldeinheiten sowohl von der effektiven Energie der Röntgenstrahlung als auch von der Streustrahlung und somit von der Form des zu untersuchenden Körperteiles abhängig. Das bewirkt, dass eine Aufnahme desselben Objektes mit zwei unterschiedlichen Geräten zu unterschiedlichen Hounsfieldwerten führt.

Um absolute Aussagen über die Verteilung der Knochendichte machen zu können, werden mitunter Phantome aus knochenäquivalentem Material bekannter Dichte zur Kalibration in Einheiten der Knochendichte verwendet. Für den Fall des CT ist ein solches Verfahren bekannt (K. Maki et al., Dentomaxillofacial Radiology, (1997) 26, 39-44), wo dem liegenden Patienten während der Aufnahme ein U-förmiges Kalibrierungsphantom auf den Kiefer gelegt wird. Die dort beschriebene Methode ist wegen des umständlich zu handhabenden und kaum reproduzierbar einzusetzenden Kalibrierungsphantoms in der Praxis allerdings nur bedingt tauglich.

Aus der US 2002/0114425 A1 ist ein Verfahren zur Bestimmung der Struktur und der Dichte von Kieferknochen im Rahmen einer herkömmlichen Röntgenprojektionsaufnahme bekannt. Um bei diesem Verfahren zu aussagekräftigen Ergebnissen zu kommen, wird das Kalibrierungsphantom, beispielsweise durch Anbringung auf einem Zahn, so angeordnet, dass es unmittelbar vor dem zu belichtenden Röntgenfilm liegt und kein weiteres absorbierendes Material zwischen Phantom und Film vorhanden ist. Schließlich ist es für die Verlässlichkeit der nach diesem Verfahren ermittelten Ergebnisse essentiell, dass die Projektion des Kalibrierphantoms als externer Standard nicht von anderen absorbierenden Strukturen, wie z.B. Knochen, überlagert werden. Auch bei dieser nur für zweidimensionale Aufnahmen geeigneten Methode ist das Kalibrierungsphantom umständlich und handhaben, wobei die Anordnung des Phantoms in späteren Aufnahmen ebenfalls nicht reproduzierbar ist.

Die US 2002/0150205 A1 offenbart ein Verfahren zur Dichtebestimmung von Knochen, wobei das um die Knochen herum angeordnete Weichgewebe durch einen Kalibrierungsvorgang eliminiert wird.

Die US 6,190,042 B1 beschreibt eine Rötgeneinrichtung für die Aufnahme eines Kieferbereichs. Diese umfasst einen Beissblock, an dem eine Filmbefestigugnsmöglichkeit vorhanden ist, einen Führungsstab zum Halten des Beissblocks und einen Befestigungsmöglichkeit für eine Röntgeneinheit.

Aufgabe der Erfindung ist es, ein Verfahren und ein System zu schaffen, das sich mit einfachen Mitteln kostengünstig umsetzen lässt und das bei einfacher und praxistauglicher Handhabung eine dreidimensionale Dichtebestimmung des Gewebes im Kieferbereich, insbesondere des Kieferknochens, mit hoher Genauigkeit und Reproduzierbarkeit ermöglicht. Weiterhin ist es Aufgabe der Erfindung, eine möglichst einfache Art der Halterung für ein Kalibrierungsphantom anzugeben.

Diese Aufgaben werden mit dem Verfahren nach Anspruch 1 und dem System nach Anspruch 6 gelöst. Vorteilhafte Ausführungsformen der Erfindung sind in den jeweiligen Unteransprüchen genannt.

Dabei ist das Kalibrierungsphantom während der Aufnahme in einer Körperhöhle des Patienten, insbesondere in die Mundhöhle, angeordnet, insbesondere eingesteckt, so dass die Bilddaten und die Kalibrierungsdaten im Rahmen derselben Aufnahme aufgenommen werden können, ohne dass es weiterer Hilfsmittel bedarf. In dieser Position ist das Phantom optimal am Ort des Geschehens platziert, so dass eine genaue Bestimmung von Gewebsdichten möglich ist.

Ein wesentlicher Grundgedanke des erfindungsgemäßen Verfahrens liegt zunächst darin, die dreidimensionale Aufnahme enthaltend den Kiefer und das Kalibrierungsphantom in aufrechter, also insbesondere in sitzender oder in stehender Haltung des Patienten zu ermöglichen. In dieser Haltung sind die Patienten einfacher handhabbar und es sind keine besonderen beweglichen Auflagen nötig, die einen erheblichen baulichen und finanziellen Aufwand für ein Aufnahmesystem bedeuten. Die erfindungsgemäße aufrechte Positionierung während der Aufnahme ermöglicht somit wesentliche kompaktere und damit anwendungsfreundlichere Aufnahmesysteme. Außerdem wird die Verwendung eines sogenannten Aufbisses als Fixierung des Patienten ermöglicht, was zu einer weiteren Reduzierung von Bewegungsartefakten führt.

Ein gewisser Vorteil gegenüber der liegenden Positionierung liegt auch darin, dass sich die Anatomie des Patienten in ihrer "natürlichen" entspannten Lage befindet, in der die Gefahr geringer ist, dass es wegen der einseitigen Belastungen zu einer Verschiebung der Knochen gegeneinander kommt, durch die eine spätere Planung beeinflusst würde. Dieser Vorteil wirkt sich insbesondere bei Aufnahmen der gegeneinander verschieblichen Kieferknochen aus.

Wie angedeutet, liegt ein weiterer wesentlicher Aspekt darin, das Kalibrierungsphantom unmittelbar in die dreidimensionale Aufnahme einzubeziehen, wobei das Kalibrierungsphantom nicht in oder am Körper des Patienten, sondern an einem Stativ in unmittelbarer Nähe des aufzunehmenden Bereichs gehalten wird. Dabei bedeutet das Merkmal "unmittelbare Nähe" zunächst nur, dass das Kalibrierungsphantom und der Schädel gemeinsam in einem Scan aufgenommen werden können. Im Idealfall ist das Kalibrierungsphantom höchstens wenige Zentimeter vom interessierenden Bereich entfernt, im Falle der Aufnahme des Kiefers insbesondere auch in der Mundhöhle, gehalten. Dabei ist das Phantom auf jeden Fall in definiertem Bezug zur bilderzeugenden Vorrichtung vorteilhafterweise aber auch in definiertem Bezug zum Patienten, beispielsweise über den Aufbiss, fixiert, so dass eine besonders gute Reproduzierbarkeit der Aufnahmen gewährleistet ist.

Aus dem Vergleich zu den vom Kalibrierungsphantom mit bekannter Dichte aufgenommenen Daten, kann auf die absoluten Dichtewerte des Gewebes geschlossen werden. Dabei liegt der besondere Vorteil des erfindungsgemäßen Verfahrens auch darin, dass es eine von Überlagerungen freie Darstellung innerhalb eines dreidimensionalen Volumens, das sowohl den Körperbereich als auch das Kalibrierungsphantom enthält, ermöglicht. Nur wegen der fehlerfreien Darstellung kann eine genaue Ermittlung beispielsweise der Knochendichten gewährleistet werden.

Ein weiterer wesentlicher Vorteil der Erfindung ist auch, dass sie zu einer reproduzierbaren Positionierung des Patienten in Bezug auf das bilderzeugende System beiträgt, was mitunter bei der Auswertung der Bilddaten von Vorteil ist und sogar die Wiederholung einzelner Aufnahmen ermöglicht. So wird erfindungsgemäß eine komfortable Lösung für eine genaue und leicht ausführbare Positionierung des Kalibrierungsphantoms relativ zum Patienten während der Aufnahme geschaffen, wobei der Patient sitzt oder steht. Mit der Erfindung wird es möglich, dentale Volumentomographen, insbesondere "Cone-Beam CTs", zu schaffen, die eine unmittelbare Knochendichtekalibration mit Hilfe von Kalibrierungsphantomen anbieten.

Um die spätere einheitliche Auswertung zu vereinfachen und den direkten Vergleich zwischen den vom Patienten und den vom Phantom aufgenommenen Bilddaten zu ermöglichen, werden die Bilddaten und die Kalibrierungsdaten in demselben Datensatz gespeichert, der damit eine dreidimensionale Repräsentation des Kieferbereichs und des zugeordneten Kalibrierungsphantoms darstellt.

Erfindungsgemäß wird zur Knochendichtebestimmung die als 'quantitative CT' (QCT) bekannte Methode eingesetzt, die insbesondere für die dreidimensionale Bildgebung vorteilhaft ist und die auf der Kalibrierung des Systems mit dem Kalibrierungsphantom beruht. Dieses hat vorteilhafterweise knochenäquivalentes Material von bekannter Dichte, beispielsweise in wasseräquivalentem Material eingebettetes Hydroxyapatit. Mit einem derartigen Phantom läßt sich bei gleichzeitiger Aufnahme des Patienten und des Phantoms durch Vergleich der Grauwerte des Knochens und des Phantoms eine zuverlässige Aussage über die absoluten Werte der Knochendichte im gescannten Knochen mit hoher Auflösung machen. Dabei nimmt die Genauigkeit der Dichtebestimmung mit der räumlichen Nähe, mit der das Kalibrierungsphantom am Kiefer plaziert wird, zu. Mit der Erfindung können Fehlerquellen, die beispielsweise durch die Strahlaufhärtung oder durch die individuelle Geometrie des zu scannenden Körpers verursacht werden und die von Ort zu Ort unterschiedlich sind, besser berücksichtigt werden.

Die tatsächliche Knochendichtebestimmung kann im einfachsten Fall durch Inaugenscheinnahme der "Aufnahme", also des aufbereiteten und als Aufnahme präsentierten Datensatzes vom Kiefer und dem zugeordneten Phantom durch den Betrachter, insbesondere den Therapeuten, geschehen. Diese Aufnahme zeigt die Strukturen gewöhnlich in Graustufen. Es ist aber auch möglich, eine farbige Darstellung des Datensatzes anzufertigen, um bestimmte Kontraste besser hervorzuheben. Durch maschinelle Auswertung können den Farben oder Graustufen absolute Dichtewerte zugeordnet und in die Darstellung integriert werden. Diese Dichtebestimmung erfolgt dabei vorteilhafterweise mit einem Auswerteprogramm, wobei dem Betrachter zur Orientierung auch absolute Dichtewerte ausgegeben werden.

Es wird das knochendichteäquivalente Material direkt in den zur Fixierung des Patienten ehedem an dem System vorhandenen Aufbiß integriert. Der Vorteil dieses Vorgehens liegt darin, dass keine zusätzliche Haltevorrichtung für das Phantom erforderlich ist. Außerdem befindet sich das Phantom in diesem Fall zwischen den Zähnen im Körper des Patienten und damit in einer besonderen Nähe zu dem interessanten Bereich.

In einer nicht beanspruchten Variante wird das Phantom von außen mittels einer gesonderten Halterung in die Nähe des Kieferbereiches des Patienten herangebracht. Die Positionierung außerhalb des Patienten hat den Vorteil, dass das Kalibrierungsphantom nicht unbedingt komplett und aufwendig desinfiziert werden braucht. Das Phantom kann mit Hilfe einer flexiblen schwenkbaren oder einer zur Patientenfixierung oder Patientenpositionierung (z.B. Aufbiss) festen Haltevorrichtung gehalten werden. Dabei kann die unmittelbare Einbettung des knochenäquivalenten Materials in die Einrichtung zur Positionierung des Patienten, beispielsweise in eine Kinnstütze, ebenfalls vorteilhaft sein. Bei der genauen und reproduzierbaren Positionierung des Phantoms entfällt die Notwendigkeit, zur Extraktion der Knochendichte-Voxelwerte Such- und Segmentierungsalgorithmen zu verwenden, da sich das knochenäquivalente Material an einer bekannten Stelle im Bildvolumen befindet. Durch die Halterung des Phantoms außerhalb des Patienten ist auch eine höhere Variabilität in der Positionierung gewährleistet.

Nachfolgend wird die Erfindung anhand der Figuren 1 bis 3 näher erklärt. Es zeigen:
- Figur 1: einen fixierten Patienten mit Kalibrierungsphantom für eine äußere Halterung, gemäß einer nicht beansüruchten Variante;
- Figur 2: ein in einem Aufbiss integriertes Kalibrierungsphantom; und
- Figur 3: ein System mit variabler Haltevorrichtung.

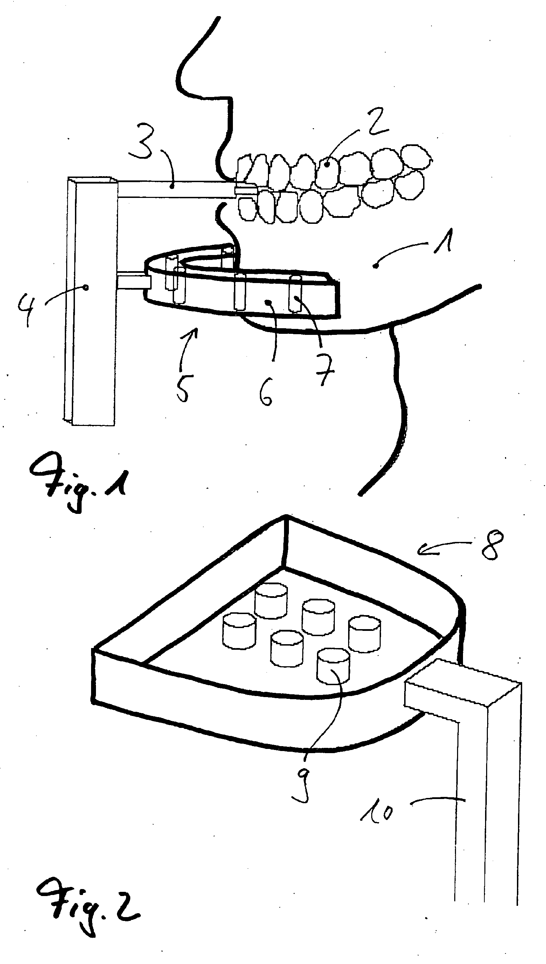

Figur 1 zeigt schematisch den Kopf, speziell den Kieferbereich 1, eines Patienten, der für die Anfertigung einer Bildsequenz mit einem Cone Beam Scanner vorbereitet ist. Mit seinen Zähnen 2 beißt der Patient auf einen Aufbiss, einer der Gebissgeometrie angepassten Schablone, um die Fixierung des Kopfes während der Sequenz zu gewährleisten. Der Aufbiss ist entsprechend über einen Träger 3 an einem Stativ 4 gehalten, wobei das Stativ 4 ortsfest ist. An dem Stativ ist ebenfalls ein in U-Form gebogenes Kalibrierungsphantom 5 befestigt, das unterhalb des Aufbisses gehalten ist. Das Kalibrierungsphantom 5 weist einen insbesondere biegsamen Mantel 6 aus weichem Kunststoff auf, der für die Röntgenstrahlung transparent ist. In dem Mantel 6 sind einzelne Zylinder 7 aus knochenäquivalentem Material, beispielsweise aus Hydroxyapatit, eingebracht, die unterschiedliche aber definierte Dichte aufweisen. Anhand der Bilddaten dieser Zylinder 7 kann später eine Kalibrierung der Gewebsdichte erfolgen. Das an dem Stativ 4 gehaltene Kalibrierungsphantom 5 läßt sich in der Höhe verstellen und mit dem biegsamen Mantel 6 an die Kontur des Patienten anpassen. Dabei kann eine unflexible Ausführung dieser Anordnung mitunter von Vorteil sein, wenn die exakte Lage des Phantoms im Bildvolumen bekannt sein muss.

Figur 2 zeigt einen Aufbiss 8, in den die Zylinder 9 aus dem knochenäquivalente Material direkt integriert sind. Diesen Aufbiss 8, der über einen Halter 10 an einem Stativ befestigt ist, umschließt der Patient zur Fixierung mit seinem Mund. Dieser integrierte Aufbiss steht ohne zusätzlichen Aufwand in jedem Bild für die Auswertung zur Verfügung. Nicht wiederverwendbare Aufbisse können Aussparungen aufweisen, in die das knochenäquivalente Material vor einem Scan eingebracht werden und später wieder entfernt werden kann. Die "Halterung" kann verworfen werden.

Mit dieser Variante des Phantoms, bei der die Knochenmaterial-Elemente mittig im Aufbiß angeordnet sind, können die störenden Einflüsse durch Metaltartefakte weiter minimiert werden.

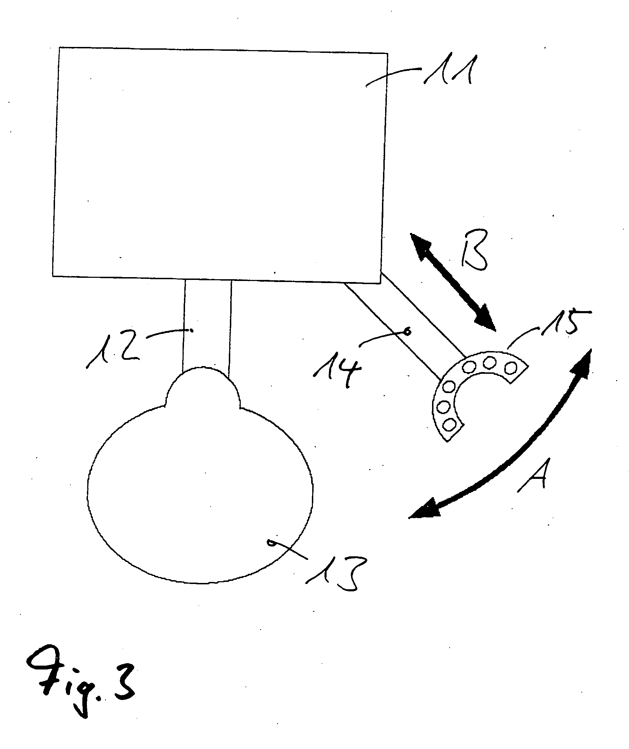

In Figur 3 sind Teile eines bildgebenden Systems gezeigt, bei dem an einer Säule 11 eine Halterung 12 für einen herkömmlichen Aufbiss angebracht ist. Die Säule gehört zu einem System aufweisend eine um den Schädel eines sitzenden oder stehenden Patienten herum bewegliche Röntgenquelle, eine Detektorvorrichtung zur Aufnahme der vom Schädel, insbesondere vom Kiefer, geschwächten Röntgenstrahlung und eine Einrichtung, insbesondere einen Aufbiss, zur Fixierung des Schädels im System. In diesem Fall ist der im Mund des Patienten 13 befindliche Aufbiss nicht zu erkennen. An der Säule ist zudem eine bewegliche Halterung 14 angebracht, das ein Kalibrierungsphantom 15 entsprechend dem aus Figur 1 trägt. Über die Halterung 14 ist das Phantom 15 verschwenkbar (Pfeil A) und axial verschieblich (Pfeil B). Auch eine vertikale Beweglichkeit ist vorgesehen. Das Stativ ist somit in mehreren Freiheitsgraden beweglich und in einer eingestellten Position festsetzbar. Über ein solches als positionierbarer Ausleger konzipiertes Stativ läßt sich das knochenäquivalente Material in Kiefernähe positionieren. Das enstprechende Phantom kann abnehmbar sein, falls es nicht benötigt wird.

## Patentansprüche

1. Verfahren zur bildlichen Darstellung und zur Bestimmung von Gewebsdichten eines Körperbereiches, insbesondere im Mund- Kiefer-Gesichtsbereich (1), eines Patienten, wobei mittels einer Bilderzeugung einerseits ein Satz dreidimensionaler Bilddaten, die den Körperbereich repräsentieren, und andererseits als dreidimensionale Bilddaten vorliegende Kalibrierungsdaten, die ein Kalibrierungsphantom (5,8,15) bekannter Dichte repräsentieren, aufgenommen werden, wobei aus einem Vergleich der Kalibrierungsdaten mit den Bilddaten auf die Gewebsdichten geschlossen wird,
wobei die Bilddaten und die Kalibrierungsdaten im Rahmen derselben Aufnahme und in aufrechter Haltung des Patienten, insbesondere im Sitzen oder im Stehen, aufgenommen werden und
wobei das Kalibrierungsphantom (5,8,15) während der Aufnahme in unmittelbarer Nähe des aufzunehmenden Körpererbereiches (1) mittels einer Halterung (4,10,14) gehalten wird, die unabhängig vom Körper des Patienten und in definiertem Bezug auf die Vorrichtung zur Bilderzeugung fixiert ist,
wobei das mittels der Halterung gehaltene Kalibrierungsphantom (8) im Körper in reproduzierbarer Position, insbesondere in der Mundhöle des Patienten, angeordnet ist.

2. Verfahren nach Anspruch 1,
**dadurch gekennzeichnet,**
**dass** der Patient in definiertem Bezug zum Kalibrierungsphantom gelagert wird.

3. Verfahren nach Anspruch 1 oder 2,
**dadurch gekennzeichnet,**
**dass** aus dem Vergleich der Kalibrierungsdaten mit den Bilddaten auf die räumliche Verteilung absoluter Dichtewerte geschlossen wird.

4. Verfahren nach einem der vorherigen Ansprüche,
**dadurch gekennzeichnet,**
**dass** der Vergleich automatisch mit einem Auswerteprogramm vorgenommen wird, wobei absolute Dichtewerte ausgegeben werden.

5. Verfahren nach einem der vorherigen Ansprüche,
**dadurch gekennzeichnet,**
**dass** die Bilddaten und die Kalibrierungsdaten mittels eines Scanners unter Verwendung der Cone-Beam Technologie aufgenommen werden.

6. System zur Durchführung eines Verfahren zur bildlichen Darstellung und zur Bestimmung von Gewebsdichten eines Körperbereiches, insbesondere im Mund- Kiefer- Gesichtsbereich (1), eines Patienten, wobei mittels einer Bilderzeugung einerseits ein Satz dreidimensionaler Bilddaten, die den Körperbereich repräsentieren, und andererseits als dreidimensionale Bilddaten vorliegende Kalibrierungsdaten, die ein Kalibrierungsphantom (5,8,15) bekannter Dichte repräsentieren, aufgenommen werden, wobei aus einem Vergleich der Kalibrierungsdaten mit den Bilddaten auf die Gewebsdichten geschlossen wird,
wobei die Bilddaten und die Kalibrierungsdaten im Rahmen derselben Aufnahme und in aufrechter Haltung des Patienten, insbesondere im Sitzen oder im Stehen, aufgenommen werden und
wobei das Kalibrierungsphantom (5,8,15) während der Aufnahme in unmittelbarer Nähe des aufzunehmenden Körpererbereiches (1) mittels einer Halterung (4,10,14) gehalten wird, die unabhängig vom Körper des Patienten und in definiertem Bezug auf die Vorrichtung zur Bilderzeugung fixiert ist, aufweisend
- eine um den Schädel eines sitzenden oder stehenden Patienten herum bewegliche Röntgenquelle,
- eine Detektorvorrichtung zur Aufnahme der vom Schädel, insbesondere vom Kiefer, geschwächten Röntgenstrahlung,
- eine Einrichtung, insbesondere einen Aufbiss, zur Fixierung des Schädels im System,
- ein zur Positionierung in unmittelbarer Nähe des Schädels an einer Halterung (4,10,12) gehaltenes Kalibrierungsphantom (5,9,15),
wobei das Kalibrierungsphantom im Körper in reproduzierbarer Position, insbesondere in der Mundhöle des Patienten, angeordnet werden kann und einen biegsamen Mantel aus weichem Kunststoff aufweist, der für Röntgenstrahlung transparent ist, wobei in dem Mantel mehrere Zylinder (7) aus knochenäquivalentem Material eingebracht sind, die unterschiedliche aber definierte Dichten aufweisen.

7. System nach Anspruch 6,
**dadurch gekennzeichnet,**
**dass** die Halterung (14) in mehreren Freiheitsgraden beweglich und in einer eingestellten Position festsetzbar ist.

8. System nach Anspruch 6,
**dadurch gekennzeichnet,**
**dass** das Kalibrierungsphantom (8) im Aufbiss integriert ist.

## Claims

1. Method for the visual presentation and for the determination of the density of tissue of a body area, in particular in the mouth-jaw-face-area (1), of a patient, wherein by means of picture generation on the one hand a set of three-dimensional image data, which represent the body area, and on the other hand calibration data, available as three-dimensional image data, which represent a calibration phantom (5,8,15) of known density, are recorded, wherein from a comparison of the calibration data and the image data the density of the tissue is gathered,
wherein the image data and the calibration data are recorded in the context of the same recording and in an upright posture of the patient, in particular during sitting or standing,
wherein during the recording the calibration phantom (5,8,15) is held in direct proximity of the body area (1) to be recorded by holding means (4,10,14), which are fixed independently from the body of the patient and in defined reference to the recording apparatus for the picture generation,
wherein the calibration phantom (8), held by the holding means, is arranged in the body in a reproducible position, in particular in the oral cavity of the patient.

2. Method according to claim 1
**characterized in**
**that** the patient is supported in a defined reference to the calibration phantom.

3. Method according to claim 1 or 2,
**characterized in**
**that** from the comparison of the calibration data and the image data the spatial distribution of absolute density values is suggested.

4. Method according to one of the preceding claims,
**characterized in**
**that** the comparison is performed automatically by an evaluation program, wherein absolute density values are issued.

5. Method according to one of the preceding claims,
**characterized in**
**that** the image data and the calibration data are recorded via a scanner using the cone beam technology.

6. System for the implementation of a method for the visual presentation and for the determination of the density of tissue of a body area, in particular in the mouth-jaw-face-area (1), of a patient, wherein by means of picture generation on the one hand a set of three-dimensional image data, which represent the body-area, and on the other hand calibration data, available as three-dimensional image data, which represent a calibration phantom (5,8,15) of known density, are recorded, wherein from a comparison of the calibration data and the image data the density of the tissue is gathered,
wherein the image data and the calibration data are recorded in the context of the same recording and in an upright posture of the patient, in particular during sitting or standing,
wherein during the recording the calibration phantom (5,8,15) is held in direct proximity of the body area (1) to be recorded by holding means (4,10,14), which are fixed independently from the body of the patient and in defined reference to the recording apparatus for the picture generation,
comprising
- a source of X-Ray, movable around the cranium of a sitting or standing patient,
- a detection device for the recording of the X-Ray radiation, debilitated by the cranium, in particular by the jaw,
- means, in particular a bite device, for fixing the cranium in the system,
- a calibration phantom (5,9,15), which is held in direct proximity of the cranium at a holding device (4,10,12),
wherein the calibration phantom can be arranged in a reproducible position, in particular in the oral cavity, and comprises a bendable casing made of soft plastic, which is transparent for X-Ray, wherein in the casing several cylinder (7) of a bone-equivalent material are inserted, which comprise different but defined densities.

7. System according to claim 6,
**characterized in**
**that** the holding means (14) are movable in several degrees of freedom and can be fastened in an adjusted position.

8. System according to claim 6,
**characterized in**
**that** the calibration phantom (8) is integrated in the bite device.

## Revendications

1. Procédé d'imagerie et de détermination de densités tissulaires d'une zone corporelle, en particulier dans la zone bucco-maxillo-faciale (1) d'un patient, dans lequel on enregistre au moyen d'une formation d'image d'une part un jeu de données d'image en trois dimensions représentant la zone corporelle et d'autre part des données de calibrage disponibles sous forme de données d'image en trois dimensions qui représentent un fantôme de calibrage (5, 8, 15) de densité connue, dans lequel en dérive les densités tissulaires d'une comparaison des données de calibrage avec les données d'image, dans lequel on enregistre les données d'image et les données de calibrage dans le cadre du même enregistrement et en position redressée du patient, en particulier en position assise ou debout, et dans lequel le fantôme de calibrage (5, 8, 15) est maintenu pendant l'enregistrement à proximité immédiate de la zone corporelle (1) à enregistrer, au moyen d'une attache (4, 10, 14) qui est fixée indépendamment du corps du patient et en relation définie par rapport au dispositif de formation d'image, dans lequel on agence le fantôme de calibrage (8) maintenu par l'attache dans une position reproductible dans le corps, en particulier dans la cavité buccale, du patient.

2. Procédé selon la revendication 1, **caractérisé en ce que** le patient est situé en relation définie par rapport au fantôme de calibrage.

3. Procédé selon la revendication 1 ou 2, **caractérisé en ce que** la répartition spatiale de valeurs de densité absolues est dérivée de la comparaison des données de calibrage avec les données d'image.

4. Procédé selon l'une quelconque des revendications précédentes, **caractérisé en ce que** la comparaison est effectuée automatiquement par un programme d'évaluation, dans lequel des valeurs de densité absolues sont délivrées.

5. Procédé selon l'une quelconque des revendications précédentes, **caractérisé en ce que** les données d'image et les données de calibrage sont enregistrées au moyen d'un scanner en utilisant la technologie « Cone Beam ».

6. Système d'exécution d'un procédé d'imagerie et de détermination de densités tissulaires d'une zone corporelle, en particulier dans la zone bucco-maxillo-faciale (1) d'un patient, dans lequel on enregistre d'une part un jeu de données d'image en trois dimensions et représentant la zone corporelle, et d'autre part des données de calibrage disponibles sous forme de données d'image en trois dimensions qui représentent un fantôme de calibrage (5, 8, 15) de densité connue, dans lequel on dérive des densités tissulaires d'une comparaison des données de calibrage avec les données d'image, dans lequel les données d'image et les données de calibrage sont enregistrées dans le cadre du même enregistrement et en position redressée du patient, en particulier en position assise ou debout, et dans lequel, pendant l'enregistrement, le fantôme de calibrage (5, 8, 15) est tenu à proximité immédiate de la zone corporelle (1) à enregistrer au moyen d'une attache (4, 10, 14) qui est fixée indépendamment du corps du patient et en relation définie par rapport au dispositif de formation d'image, comprenant
- une source de rayons X mobile autour du crâne d'un patient assis ou debout,
- un dispositif de détection pour enregistrer le rayonnement X atténué par le crâne, en particulier la mâchoire,
- un dispositif, en particulier une plaque d'occlusion, pour fixer le crâne dans le système,
- un fantôme de calibrage (5, 9, 15) maintenu au niveau d'une attache (4, 10, 12) à proximité immédiate du crâne en vue du positionnement,
dans lequel le fantôme de calibrage peut être agencé dans une position reproductible dans le corps, en particulier dans la cavité buccale du patient, et présente une gaine en matière plastique souple transparente au rayonnement X, dans lequel plusieurs cylindres (7) en matériau équivalent os sont introduits dans la gaine et présentent des densités différentes mais définies.

7. Système selon la revendication 6, **caractérisé en ce que** l'attache (14) est mobile selon plusieurs degrés de liberté et peut être fixée dans une position réglée.

8. Système selon la revendication 6, **caractérisé en ce que** le fantôme de calibrage (8) est intégré dans la plaque d'occlusion.
